(19) Europäisches Patentamt
European Patent Office
Office européen des brevets

(11) Veröffentlichungsnummer: **0 019 148**
**B1**

(12) **EUROPÄISCHE PATENTSCHRIFT**

(45) Veröffentlichungstag der Patentschrift: **20.07.83**

(51) Int. Cl.³: **C 07 D 498/04,** A 61 K 31/42, C 12 P 17/18 // (C07D498/04, 263/00, 205/00)

(21) Anmeldenummer: **80102351.6**

(22) Anmeldetag: **30.04.80**

(54) **Antibiotikum zur Unterdrückung des Wachstums von Mikroorganismen sowie dessen Herstellung durch Fermentation.**

(30) Priorität: **04.05.79 US 35884**

(43) Veröffentlichungstag der Anmeldung: **26.11.80 Patentblatt 80/24**

(45) Bekanntmachung des Hinweises auf die Patenterteilung: **20.07.83 Patentblatt 83/29**

(84) Benannte Vertragsstaaten: **AT BE CH DE FR GB IT LI NL**

(56) Entgegenhaltungen:
**DE-A-2 517 316**
**DE-A-2 702 091**
**DE-A-2 725 690**

**Chemical Abstracts Band 91, Nr. 19, 5. November 1979 Columbus, Ohio, USA D. BROWN et al. «Structures of three novel Beta-lactams isolated from Streptomyces clavuligerus» Seite 305, Spalte 1, Abstract Nr. 153 931h**

(73) Patentinhaber: **F. HOFFMANN-LA ROCHE & CO. Aktiengesellschaft, CH-4002 Basel (CH)**

(72) Erfinder: **Kellett, Martha, 121 Hillside Avenue, Nutley, N.J. (US)**
Erfinder: **Pruess, David, 12 Brookside Terrace, North Caldwell, N.J. (US)**
Erfinder: **Scannell, James P., 4 Canterbury Drive, North Caldwell, N.J. (US)**

(74) Vertreter: **Wuesthoff, Franz, Dr.-Ing. et al, Patentanwälte Wuesthoff -v. Pechmann-Behrens-Goetz Schweigerstrasse 2, D-8000 München 90 (DE)**

**EP 0 019 148 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Antibiotikum zur Unterdrückung des Wachstums von Mikroorganismen sowie dessen Herstellung durch Fermentation

Die vorliegende Erfindung betrifft ein Antibiotikum der Formel

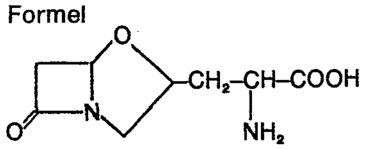

I

Das in reiner Form vorliegende Antibiotikum zeigt antimikrobielle Aktivität in vitro gegen eine Mehrzahl von Mikroorganismen und ist deshalb nützlich in Waschlösungen, die für hygienische Zwecke verwendet werden, wie beispielsweise das Waschen von Händen und die Reinigung von Einrichtungen, Böden und Mobiliar von kontaminierten Räumen und Laboratorien.

Es ist ebenso nützlich, um das Wachstum von empfindlichen Mikroorganismen in Plattenversuchen und anderen mikrobiologischen Medien zu unterdrücken.

Die neue antibiotisch wirksame Verbindung der Formel I wird chemisch als 3-(7-Oxo-4-oxa-1-azabicyclo[3.2.0]hept-3-yl)alanin oder 2-(3-Alanyl)clavam bezeichnet. Erfindungsgemäss kann sie hergestellt werden, indem man einen antibiotika-produzierenden Stamm von Streptomyces clavuligerus in einem wässrigen Nährmedium, das assimilierbare Kohlenstoff- und Stickstoffquellen enthält, unter submersen, aeroben Bedingungen kultiviert, bis der Kulturansatz eine erhebliche antibiotische Aktivität zeigt und anschliessend die so produzierte Verbindung der Formel I aus dem obengenannten wässrigen Medium isoliert.

Streptomyces clavuligerus ist ein bekannter Mikroorganismus, wurde er doch schon früher für die Herstellung der β-Lactamase-Inhibitoren Clavulansäure (belgisches Patent Nr. 827 926 bzw. DE-A-2 517 316) und β-Hydroxy-propionyl-clavulansäure (DE-A-2 708 047) und von antifungisch wirksamen Verbindungen der Formel

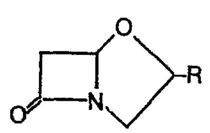

II

worin R $-CH_2OH$, $-CH_2-O\overset{\overset{O}{\|}}{C}H$ oder $-COOH$ bedeutet (DE-A-2 725 690 und südafrikanisches Patent Nr. 773 414), verwendet.

Bekannt sind ebenfalls solche Verbindungen der Formel II, worin R ein Wasserstoffatom, ein Alkenyl- oder Epoxidrest mit 2 bis 4 Kohlenstoffatomen oder ein Alkylrest mit 1 bis 4 Kohlenstoffatomen ist, der mit 1 oder 2 Halogenatomen, gegebenenfalls acylierten oder mit Resten mit 1 bis 7 Kohlenstoffatomen verätherten Hydroxylgruppen, Azido- oder Phthalamidoresten substituiert sein kann, oder ein Rest der Formeln $SR^1$, $SOR^1$, $SO_2R^1$ oder $SeR^1$ ist, in denen $R^1$ ein Arylrest ist (DE-A-2 702 091).

Eine Verbindung vom Typus der Formel II, worin R eine 3-Alanylgruppe darstellt, d.h. die Verbindung der Formel I, wurde jedoch bisher nicht beschrieben.

Subkulturen des Mikroorganismus Streptomyces clavuligerus sind im United States Department of Agriculture, Agricultural Research Service, in Northern Regional Research Laboratories (NRRL), Peoria, Illinois, USA, unter der Identifikationsnummer NRRL 3585 und in der American Type Culture Collection (ATCC), Rockville, Maryland, USA, unter der Identifikationsnummer ATCC 27064 deponiert. Zusätzlich wurde eine Subkultur der deponierten Kultur NRRL 3585 bei ATCC hinterlegt und erhielt die Identifikationsnummer ATCC 31620. Die antibiotisch wirksame Verbindung der Formel I kann durch Fermentation all dieser deponierten Kulturen hergestellt werden, darüber hinaus aber auch durch Fermentation von Subkulturen und Mutanten davon. Tatsächlich kann die antibiotisch wirksame Verbindung der Formel I durch jeden antibiotica-produzierenden Stamm von Streptomyces clavuligerus produziert werden. Morphologische Daten für die deponierten Kulturen NRRL 3585 und ATCC 27064 sind in der belgischen Patentschrift No. 827 926 beschrieben.

Eine Streptomyces clavuligerus enthaltende Gärbrühe wird durch Beimpfung eines geeigneten Nährbodens mit Sporen oder Mycel des Clavulansäure-Antibiotikum erzeugenden Mikroorganismus und anschliessende Kultivierung unter aeroben Bedingungen hergestellt. Die Kultivierung an einem festen Nährboden ist möglich, doch wird für die Herstellung grössere Mengen des Antibiotikums ein flüssiges Medium bevorzugt. Die Gärtemperatur kann in einem weiten Bereich, 20–35 °C, variiert werden, jedoch sind Temperaturen von 26–30 °C und im wesentlichen neutrales pH bevorzugt. Für die submerse, aerobe Fermentation kann das Nährmedium als Kohlenstoffquelle ein im Handel erhältliches Glycerinöl oder ein Kohlenhydrat, wie z.B. Glycerin, Maltose, Lactose, Dextrin, Stärke, usw., in reinem oder rohem Zustand enthalten; als Stickstoffquelle kommen organische Materialien, wie Sojabohnenmehl, lösliche Rückstände aus der Brennerei, Erdnussmehl, Baumwollsamenmehl, Fleischextrakt, Pepton, Fischmehl, Hefeextrakt, Maisquellwasser, usw., in Betracht. Falls erwünscht, können auch anorganische Stickstoffquellen, wie z.B. Nitrate und Ammoniumsalze und Mineralsalze, wie z.B. Ammoniumsulfat, Magnesiumsulfat u.dgl. verwendet werden. Das Nährmedium kann auch Natriumchlorid, Kaliumchlorid, Kaliumphosphat u.dgl. und Puffersubstanzen, wie z.B. Natriumcitrat, Calciumcarbonat und Phosphate, und Spuren von Schwermetallsalzen enthalten. Wird die Fermentation unter belüfteten, submersen Bedingungen durchgeführt, verwendet man vorzugsweise ein Schaumbekämpfungsmittel, wie flüssiges Paraffin, fette Öle oder Silikonverbindungen. Mehr als eine Art von Kohlenstoffquellen, Stickstoffquelle bzw. Schaumbekämpfungsmittel können für die

Herstellung des Antibiotikums verwendet werden. Im allgemeinen wird die Kultivierung fortgesetzt, bis die Konzentration des Antibiotikums im Nährmedium mindestens 40 µg/ml beträgt.

Die folgenden Beispiele erläutern die vorliegende Erfindung ohne sie einzuschränken.

Beispiel 1

Streptomyces clavuligerus NRRL 3585 wird auf Stärke/Casein-Agarschrägkulturen aufbewahrt. Ein Teil der Schrägkultur-Züchtung wird verwendet, um 100 ml einer $TS^+$-Gärlösung (wie weiter unten definiert) in einem 500 ml-Erlenmeyerkolben zu beimpfen. Anschliessend inkubiert man bei 28 °C während 2 Tagen auf einer Schüttelmaschine, gibt 20 ml einer sterilen 50%igen Glycerinlösung hinzu, füllt je 4–6 ml der erhaltenen Mischung in 10 ml-Probegläser mit Schraubdekkel-Verschluss ab, friert sofort in einem Trokkeneisbad ein und bewahrt die Proben bei –70 °C auf. Das Impfsubstrat wird hergestellt, indem man den Inhalt der Probegläser schmilzt und je 10 ml dieser Kultur in zwei 6-Liter-Erlenmeyerkolben gibt, die je 2 l einer $TS^+$-Mediums folgender Zusammensetzung enthalten:

| | |
|---|---|
| Trypticace Soy Broth dehydrated | 30 g |
| Glycerin | 10 g |
| destilliertes Wasser | 1 l |
| ohne pH-Einstellung | |

Die 6-Liter-Kolben werden bei 28 °C während 3 Tagen auf einer Schüttelmaschine inkubiert. Gute Resultate erhält man bei Inkubationszeiten von 1 Tag bis 4 Tage. Die 4 l Vorkultur wird anschliessend zur Beimpfung eines 230-Liter-Substratansatzes der folgenden Zusammensetzung verwendet:

| | |
|---|---|
| Glycerin | 20 g |
| fettarmes Sojabohnenmehl | 15 g |
| $K_2HPO_4$ | 1 g |
| $CaCl_2 \cdot 6H_2O$ | 10 mg |
| Schaumbekämpfungsmittel | 0,1 g |
| Leitungswasser | 1 l |

Der pH wird vor dem Sterilisieren mit 5n Schwefelsäure auf 7,0 eingestellt.

Beispiel 2

Das Impfsubstrat wird hergestellt, indem man 2 ml der in Beispiel 1 beschriebenen Kultur auftaut und zu 100 ml $TS^+$-Gärlösung (wie in Beispiel 1 definiert) in einem 500 ml-Kolben gibt, einen Tag bei 28 °C schüttelt, anschliessend 6-Liter-Erlenmeyerkolben mit je 20 ml dieser Kultur beimpft und dann wie in Beispiel 1 weiterfährt.

Beispiel 3

Die Fermentation in einem 400 ml rostfreien Stahlfermenter wird bei 28 °C unter Rühren (150 U./min) durchgeführt. Die Belüftung wird zwischen 85 und 140 l/Min. variiert, so dass die Konzentration des gelösten Sauerstoffs mehr als 50% der Sättigungskonzentration beträgt. Der Messdruck im Fermenter beträgt 0,35 kg/cm². Der pH wird mit 5n Schwefelsäure oder 5n Natronlauge, je nachdem was nötig ist, zwischen 6,5 und 7,5 eingestellt.

Die jeweilige Konzentration des Antibiotikums in entnommenen Proben wird durch Ermittlung der Wirkung gegen Bacillus TA 1283 B (ATCC 27860) auf dem Minimalagar von B.B. Davies und E.S. Mingioli bestimmt (J. Bact. 60, 17–28, 1950).

Nach Inkubation während 4 Tagen werden 190 l Gärlösung geerntet. 30–40 l hat man während der Fermentation probenweise entnommen. Man kühlt die Gärlösung (pH 7,2) auf 20 °C ab, gibt 1900 g Aktivkohle hinzu und rührt die Mischung während 15 Minuten. Der pH wird mit 100 ml 5n Schwefelsäure auf 6,5 eingestellt, anschliessend fügt man 6,8 kg Kieselgur hinzu und filtriert mit einem Rotations-Vakuumfilter vom Mycel ab. Der Filterkuchen wird mit 22 l Leitungswasser gewaschen.

Filtrat und Waschwasser werden vereinigt (210 l, pH 7,7), der pH wird mit 5n Schwefelsäure auf 7,3 eingestellt. Die Lösung wird auf 22 l eingeengt.

Das Konzentrat wird mit einer Geschwindigkeit von 1,5 l/Min. auf eine Säule (30 cm Durchmesser) gegeben, die 45 kg Kationenaustauscher (Amberlite®XAD-2, Warenzeichen von Rohm und Haas Inc., USA, für ein nichtfunktionelles Polystyrolharz mit 2% Divinylbenzolvernetzung).

Die Aktivität wird mit 500 ml entionisiertem Wasser vom Harz eluiert. Nach dem Auftreten von Salzen im Eluat werden Fraktionen zu 20 l gesammelt. Aufgrund von Versuchsergebnissen werden die Fraktionen 2, 4 und 5 auf Endvolumen von 2,3 l, 2,2 l und 2,7 l eingeengt; die Fraktionen 6 und 7 werden vereinigt und auf 2,3 l eingeengt. Fraktion 3 wird in einem Gefriertrockner lyophilisiert und ergibt 175 g eines festen Stoffes. Fraktion 3 enthält den Grossteil des Antibiotikums, dennoch sind die spezifischen Aktivitäten der Fraktionen 4–7 höher und es zeigt sich, dass letzteres Material für die weitere Reinigung geeigneter ist.

Beispiel 4

10 g Material aus Fraktion 4 der Amberlite®-XAD-2-Kolonne werden in etwa 30 ml Wasser gelöst und filtriert. Die so erhaltene klare, gelb-braune Lösung (6,5 g Festkörper, 38 ml Endvolumen) wird in einem Flüssigchromatographen an einer modifiziertes Kieselgel enthaltenden Säule chromatographiert, unter Eluieren mit Wasser. Nach einem Vorlauf von 400 ml werden Fraktionen zu 100 ml gesammelt und biologisch untersucht. Man vereinigt die aktivsten Fraktionen, lyophilisiert sie und erhält so 910 mg eines festen Stoffes.

900 mg dieses Festkörpers werden in Wasser gelöst und an 6 in Serie geschalteten modifiziertes Kieselgel enthaltenden Säulen (60 × 1 cm), unter Eluieren mit Wasser, chromatographiert. Nach einem Vorlauf von etwa 90 ml werden Fraktionen zu 5 ml gesammelt. Die biologisch aktivsten Fraktionen (Nrn. 28–31) ergeben nach Vereinigen und Lyophilisieren 105 mg gelblichen Festkörper. 100 mg dieses Stoffes werden in 1 ml Wasser gelöst, mit 1 ml Methanol verdünnt und auf eine Dextran-Säule (Sephadex®LH-20; 50 × 2,5 cm; Warenzeichen von Pharmacia Fine Chemicals AB, Sweden, für ein hydroxypropyliertes, vernetztes

Dextrangel) gegeben. Nach Eluieren mit 4 Bedvolumen Methanol/Wasser (1:1) werden 50 Fraktionen zu 3 ml gesammelt. Nach Vereinigen der aktivsten Fraktionen (Nrn. 35–41) wird auf ein kleines Volumen eingeengt und langsam Äthanol zugegeben. Nach Abschrecken der Lösung auf etwa 4 °C erhält man 17 mg kristallines Material, aus der Mutterlauge erhält man noch eine zweite Portion von 8 mg.

Beispiel 5

10 g lyophilisiertes Material aus den Fraktionen 5–7 der Amberlite®XAD-2-Säule (Beispiel 3) wird in 50 ml Methanol/Wasser (1:1) aufgeschlämmt und filtriert, unter Nachspülen mit 25 ml desselben Lösungsmittelgemisches. Die vereinigten Filtrate werden auf eine Säule (50 × 15,5 cm), welche 9 l Dextran-Gel (Sephadex®LH-20) in Methanol/Wasser (1:1) enthält, gegeben. Das Gel wird bei 5 °C und einer Flussrate von 800 ml/Stunde mit demselben Lösungsmittelgemisch eluiert. Die aktivste Fraktion, bei einem Elutionsvolumen von 6,3–6,9 l erhalten, wird unter vermindertem Druck bei 30 °C auf ein Volumen von 25 ml eingeengt.

18 ml dieser Lösung, die 900 mg Festkörper enthält, wird in einem Flüssigchromatographen an einer modifiziertes Kieselgel enthaltende Säule chromatographiert. Nach Durchlauf des Bedvolumens (400 ml) werden 20 Fraktionen zu je 80 ml gesammelt und biologisch untersucht. Durch Vereinigen und Lyophilisieren der aktivsten Fraktionen (Nrn. 6–9) erhält man 178 mg Festkörper, den man in 1 ml Wasser löst, mit 1 ml Methanol verdünnt und auf eine Dextran-Säule (Sephadex®LH-20; 50 × 2,5 cm) gibt. Nach Eluieren mit 4 Bedvolument Methanol/Wasser (1:1) werden die aktivsten Fraktionen vereinigt, im Vakuum bei 30 °C eingeengt und mit Äthanol versetzt. Nach Abkühlen auf 4 °C können 20 mg kristallines Material abfiltriert werden. Beim Stehenlassen über Nacht bei 4 °C fallen weitere 20 mg Material an.

Beispiel 6

2 ml einer wässrigen Lösung von 760 mg rohem Antibiotikum, erhalten aus der ersten Sephadex®LH-20-Säule (Beispiel 5), wird an 6 in Serie geschalteten modifiziertes Kieselgel enthaltenden Säulen (60 × 1 cm) unter Eluieren mit Wasser chromatographiert. Nach Durchlauf des Bedvolumens (90 ml) sammelt man Fraktionen zu 5 ml und untersucht sie biologisch. Die aktivsten Fraktionen (Nrn. 31–35) werden vereinigt und im Vakuum bei 30 °C auf ein Volumen von ca. 1 ml eingeengt. Durch Zugabe von Äthanol und Abschrecken auf 4 °C erhält man 22 mg kristallines Antibiotikum, $[\alpha]^{25} = -137{,}6$ (c = 0,7; $H_2O$).

Beispiel 7

17 g lyophilisiertes Material aus Fraktion 4 der früher beschriebenen Amberlite®XAD-2-Säule werden in 80 ml Wasser vom pH 6,6 aufgeschlämmt. Von unlöslichem Material, vorwiegend Aktivkohle, wird abfiltriert, unter Nachspülen mit Wasser. Filtrat und Waschwasser werden vereinigt (insgesamt 100 ml) und auf eine Säule (50 × 4,2 cm), die 700 ml Kationenaustauscher (Bio-Rod®AG 50W X-4, 100-200 Mesh; Warenzeichen von Bio-Rod Inc., USA, für ein stark saures Styrol/Divinylbenzol-Copolymer mit Sulfonsäuregruppen) in der $Na^+$-Form enthält, aufgetragen. Die Säule wird bei 5 °C mit destilliertem Wasser gewaschen und eluiert. Die aktivste Fraktion erhält man bei einem Elutionsvolumen von 400–500 ml nach Erscheinen von nichtadsorbiertem Material im Eluat. Die aktive Fraktion wird auf 2 ml eingeengt. 1 ml davon wird mit 2 ml Äthanol versetzt, dabei fallen 32 mg kristallines Material an. Der Rest der aktiven Fraktion (1 ml) wird lyophilisiert; aus dem Rückstand (48 mg) erhält man noch weiteres, kristallines Material. Smp. 247–265 °C (langsame Zersetzung).

Beispiel 8

Die in vitro antimikrobielle Aktivität des Antibiotikums (minimale Hemmungskonzentration) wird mittels der Agar-Diffusionsmethode bestimmt. Die Resultate in µ/ml sind wie folgt:

| | | Deponierungs-Nr. | MIC (µg/ml) Antibiotic Medium Versuchs-Agar[1] | Davis Minimal-Agar |
|---|---|---|---|---|
| Gram-negative Stäbchen | Pseudomonas aeruginosa | ATCC 8709 | >1000 | >1000 |
| | Proteus vulgaris | ATCC 6380 | >1000 | * |
| | Escherichia coli | ATCC 27856 | >1000 | 1000 |
| | Klebsiella pneumoniae | ATCC 27858 | >1000 | 62,5 |
| | Serratia marcescens | ATCC 27857 | >1000 | >500 |
| | Serratia sp. | ATCC 93 | >1000 | 500 |
| | Acinetobacter calcoaceticus | ATCC 10153 | >1000 | >500 |
| Gram-positive Kokken | Staphylococcus aureus | ATCC 6538 P | >1000 | * |
| | Sarcina lutea | ATCC 9341 | >1000 | >500 |
| | Streptococcus faecium | ATCC 8043 | >1000 | * |

Fortsetzung

| | | Deponierungs-Nr. | MIC (µg/ml) Antibiotic Medium Versuchs-Agar[1] | Davis Minimal-Agar |
|---|---|---|---|---|
| Gram-positive Stäbchen | Bacillus megaterium | ATCC 8011 | >1000 | >500 |
| | Bacillus sp. E | ATCC 27859 | >1000 | * |
| | Bacillus subtilis | NRRL 558 | >1000 | 0,25 |
| | Bacillus sp. TA | ATCC 27860 | >1000 | 0,03 |
| Gram-positive Filamente | Mycobacterium phlei | ATCC 355 | >1000 | >500 |
| | Streptomyces cellulosae | ATCC 3313 | >1000 | >500 |
| Schimmelpilze | Paecilomyces varioti | ATCC 26820 | 250 | 15,7 |
| | Penicillium digitatum | ATCC 26821 | >1000 | * |
| Hefen | Candida albicans | NRRL 477 | >1000 | 500 |
| | Saccharomyces cerevisiae | ATCC 4226 | >1000 | 125 |

* Microorganismus wächst nicht
[1] Zusammensetzung (g/l): Pepton 6,0, Pankreas-Auszug von Kasein 4,0, Hefeextrakt 3,0, Fleischextrakt 1,5, Glucose 1,0, Agar 15,0

**Patentansprüche für die Vertragsstaaten: BE, CH, DE, FR, GB, IT, LI, NL**

1. Antibiotikum der Formel

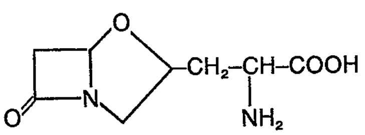

I

2. Antibiotikum gemäss Anspruch 1 in gereinigter Form.

3. Antibiotikum gemäss Anspruch 1 oder 2 als Wirkstoff zur Unterdrückung des Wachstums von Mikroorganismen.

4. Antibiotikum gemäss Anspruch 1 oder 2 als Wirkstoff in Waschlösungen für hygienische Zwekke zur Unterdrückung des Wachstums von Mikroorganismen.

5. Verfahren zur Herstellung des Antibiotikums gemäss Anspruch 1 oder 2, dadurch gekennzeichnet, dass man eine Subkultur von Streptomyces clavuligerus NRRL 3585 (ATCC 27 064, ATCC 31 620) in einem wässrigen Nährmedium, das assimilierbare Kohlenstoff- und Stickstoffquellen enthält, unter submersen, aeroben Bedingungen kultiviert, bis der Kulturansatz eine erhebliche antibiotische Aktivität gegen Bacillus TA 1283 B (ATCC 27 860) auf dem Minimalagar von B.B. Davies und E.S. Mingioli:

| | |
|---|---|
| $K_2HPO_4$ | 7.0 |
| $KH_2PO_4$ | 3.0 |
| $Na_3$-citrat · $3H_2O$ | 0.5 |
| $MgSO_4 \cdot 7H_2O$ | 0.1 |
| $(NH_4)_2SO_4$ | 1.0 |
| Glucose | 2.0 |
| Agar | 15.0 |
| $H_2O$ | 1000 |
| pH | 7.0 |

zeigt, und anschliessend die so produzierte Verbindung der Formel I gemäss Anspruch 1 aus dem obengenannten wässrigen Medium durch Fraktionierung isoliert, wobei diejenigen Fraktionen isoliert werden, welche gegen den erwähnten Bacillus-Stamm auf dem erwähnten Minimalagar antibiotisch wirksam sind.

**Patentansprüche für den Vertragsstaat: AT**

1. Verfahren zur Herstellung des Antibiotikums der Formel

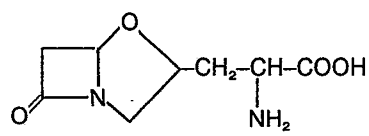

I

dadurch gekennzeichnet, dass man eine Subkultur von Streptomyces clavuligerus NRRL 3585 (ATCC 27 064, ATCC 31 620) in einem wässrigen Nährmedium, das assimilierbare Kohlenstoff- und Stickstoffquellen enthält, unter submersen, aeroben Bedingungen kultiviert, bis der Kulturansatz eine erhebliche antibiotische Aktivität gegen Bacillus TA 1283 B (ATCC 27 860) auf dem Minimalagar von B.B. Davies und E.S. Mingioli:

| | |
|---|---|
| $K_2HPO_4$ | 7.0 |
| $KH_2PO_4$ | 3.0 |
| $Na_3$-citrat · $3H_2O$ | 0.5 |
| $MgSO_4 \cdot 7H_2O$ | 0.1 |
| $(NH_4)_2SO_4$ | 1.0 |
| Glucose | 2.0 |
| Agar | 15.0 |
| $H_2O$ | 1000 |
| pH | 7.0 |

zeigt, und anschliessend die so produzierte Verbindung der Formel I aus dem obengenannten wässrigen Medium durch Fraktionierung isoliert, wobei diejenigen Fraktionen isoliert werden, welche gegen den erwähnten Bacillus-Stamm auf dem erwähnten Minimalagar antibiotisch wirksam sind.

2. Verfahren nach Anspruch 1, dadurch gekennzeichnet, dass man die Kultivierung bei 20–35 °C, insbesondere bei 26–30 °C, und im wesentlichen neutrales pH durchführt.

3. Verfahren nach Anspruch 1 oder 2, dadurch gekennzeichnet, dass man das Antibiotikum der Formel I aus der Kulturansatz in der Weise isoliert, dass man letzteren ansäuert und filtriert und das Filtrat chromatographisch reinigt.

**Claims for the contracting states: BE, CH, DE, FR, GB, IT, LI, NL**

1. Antibiotic of the formula

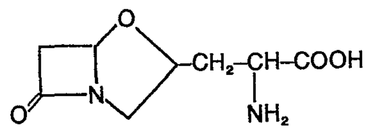

I

2. Antibiotic in accordance with claim 1 in purified form.

3. Antibiotic in accordance with claim 1 or 2 as an active substance for suppressing the growth of microorganisms.

4. Antibiotic in accordance with claim 1 or 2 as an active substance in washing solutions for hygienic purposes for suppressing the growth of microorganisms.

5. Process for the manufacture of the antibiotic in accordance with claim 1 or 2, characterized by cultivating a subculture of Streptomyces clavuligerus NRRL 3585 (ATCC 27 064, ATCC 31 620) in an aqueous nutrient medium, which contains assimilable carbon and nitrogen sources, under submersed aerobic conditions until the culture batch exhibits a considerable antibiotic activity against Bacillus TA 1283 B (ATCC 27 860) on the minimal agar of B.B. Davies and E.S. Mingioli:

| | |
|---|---|
| $K_2HPO_4$ | 7.0 |
| $KH_2PO_4$ | 3.0 |
| $Na_3$ citrate $\cdot 3H_2O$ | 0.5 |
| $MgSO_4 \cdot 7H_2O$ | 0.1 |
| $(NH_4)_2SO_4$ | 1.0 |
| glucose | 2.0 |
| agar | 15.0 |
| $H_2O$ | 1000 |
| pH | 7.0 |

and subsequently isolating the thus-produced compound of formula I in accordance with claim 1 from the aforementioned aqueous medium by fractionation, there being isolated those fractions which have antibiotic activity against the mentioned Bacillus strain on the mentioned minimal agar.

**Claims for the contracting state: AT**

1. Process for the manufacture of the antibiotic of the formula

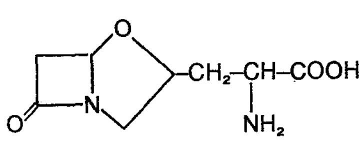

I

characterized by cultivating a subculture of Streptomyces clavuligerus NRRL 3585 (ATCC 27 064, ATCC 31 620) in an aqueous nutrient medium, which contains assimilable carbon and nitrogen sources, under submersed, aerobic conditions until the culture batch exhibits a considerable antibiotic activity against Bacillus TA 1283 B (ATCC 27 860) on the minimal agar of B.B. Davies and E.S. Mingioli:

| | |
|---|---|
| $K_2HPO_4$ | 7.0 |
| $KH_2PO_4$ | 3.0 |
| $Na_3$ citrate $\cdot 3H_2O$ | 0.5 |
| $MgSO_4 \cdot 7H_2O$ | 0.1 |
| $(NH_4)_2SO_4$ | 1.0 |
| glucose | 2.0 |
| agar | 15.0 |
| $H_2O$ | 1000 |
| pH | 7.0 |

and subsequently isolating the thus-produced compound of formula I from the aforementioned aqueous medium by fractionation, there being isolated those fractions which have antibiotic activity against the mentioned Bacillus strain on the mentioned minimal agar.

2. Process according to claim 1, characterized in that the cultivation is carried out at 20–35 °C, especially at 26–30 °C, and in substantially neutral pH.

3. Process according to claim 1 or 2, characterized in that the antibiotic of formula I is isolated from the culture batch by acidifying and filtering the latter and purifying the filtrate chromatographically.

**Revendications pour les Etats contractants: BE, CH, DE, FR, GB, IT, LI, NL**

1. Antibiotique de formule

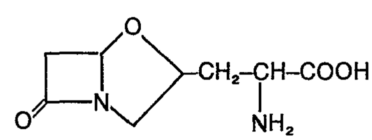

I

2. Antibiotique selon la revendication 1 sous forme purifiée.

3. Antibiotique selon l'une des revendications 1 ou 2 comme substance active pour réprimer la croissance de microorganismes.

4. Antibiotique selon l'une des revendications 1 ou 2 comme matière active dans les solutions de lavage dans des buts hygiéniques pour réprimer la croissance de microorganismes.

5. Procédé de préparation de l'antibiotique selon l'une des revendications 1 ou 2, caractérisé en ce qu'on cultive une sous-culture de Streptomyces clavuligerus NRRL 3585 (ATCC 27 064, ATCC 31 620) dans un milieu nutritif aqueux, qui contient des sources de carbone et d'azote assimilables, dans des conditions aérobies en gélose profonde, jusqu'à ce que la culture présente une notable activité antibiotique contre Bacillus TA 1283 B (ATCC 27 860) sur la gélose minimale de B.B. Davies et E.S. Mingioli:

| | |
|---|---|
| $K_2HPO_4$ | 7,0 |
| $KH_2PO_4$ | 3,0 |
| Citrate de $Na_3 \cdot 3H_2O$ | 0,5 |
| $MgSO_4 \cdot 7H_2O$ | 0,1 |
| $(NH_4)_2SO_4$ | 1,0 |
| Glucose | 2,0 |
| Gélose | 15,0 |
| $H_2O$ | 1000 |
| pH | 7,0 |

puis en ce qu'on isole par fractionnement du milieu aqueux mentionné ci-dessus le composé de formule I selon la revendication 1 ainsi produit, en isolant les fractions qui sont actives comme antibiotique contre la souche de bacille mentionnée sur la gélose minimale mentionnée.

**Revendications pour l'Etat contractant: AT**

1. Procédé de préparation de l'antibiotique de formule:

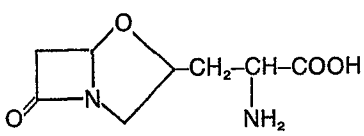

I

caractérisé en ce qu'on cultive une sous-culture de Streptomyces clavuligerus NRRL 3585 (ATCC 27 064, ATCC 31 620) dans un milieu nutritif aqueux qui contient des sources de carbone et d'azote assimilables, dans des conditions aérobies en glélose profonde, jusqu'à ce que le dépôt de culture présente une activité antibiotique notable contre Bacillus TA 1283 B (ATCC 27 860) sur gélose minimale de B.B. Davies et E.S. Mingioli:

| | |
|---|---|
| $K_2HPO_4$ | 7,0 |
| $KH_2PO_4$ | 3,0 |
| Citrate de $Na_3 \cdot 3H_2O$ | 0,5 |
| $MgSO_4 \cdot 7H_2O$ | 0,1 |
| $(NH_4)_2SO_4$ | 1,0 |
| Glucose | 2,0 |
| Gélose | 15,0 |
| $H_2O$ | 1000 |
| pH | 7,0 |

puis en ce qu'on isole par fractionnement du milieu aqueux mentionné ci-dessus le composé de formule I ainsi produit, en isolant les fractions qui sont actives comme antibiotiques contre la souche de Bacille mentionnée sur la gélose minimale mentionnée.

2. Procédé selon la revendication 1, caractérisé en ce qu'on conduit la culture à 20–35 °C, en particulier à 26–30 °C, et à pH pratiquement neutre.

3. Procédé selon l'une des revendications 1 ou 2, caractérisé en ce qu'on isole l'antibiotique de formule I à partir du dépôt de culture de manière à acidifier ce dernier et à le filtrer, et à purifier le filtrat par chromatographie.